# EUROPÄISCHE PATENTSCHRIFT

(11) **EP 2 979 094 B1**
(45) Veröffentlichungstag und Bekanntmachung des Hinweises auf die Patenterteilung: **29.08.2018**
(21) Anmeldenummer: 14720499.4
(22) Anmeldetag: 28.03.2014
(51) Int. Cl.: G01N 33/564, C07K 14/00, C07K 14/47

(54) **VERFAHREN ZUR DIAGNOSE VON NEUROMYELITIS OPTICA**
METHOD FOR THE DIAGNOSIS OF NEUROMYELITIS OPTICA
PROCÉDÉ DE DIAGNOSTIC DE LA NEUROMYÉLITE OPTIQUE

(30) Priorität: 28.03.2013 EP 13161822
(43) Veröffentlichungstag der Anmeldung: 03.02.2016
(73) Patentinhaber: Protagen AG, 44227 Dortmund (DE)
(72) Erfinder: GÖHLER, Heike, 44797 Bochum (DE); RENGERS, Petra, 48161 Münster (DE); MÜLLNER, Stefan, 40764 Langenfeld (DE); LÜKING, Angelika, 44892 Bochum (DE)
(74) Vertreter: Simandi, Claus
(86) Internationale Anmeldenummer: PCT/EP2014/056383
(87) Internationale Veröffentlichungsnummer: WO 2014/154907

(56) Entgegenhaltungen:
- WO-A1-2012/149259
- WO-A2-2005/051178
- WO-A2-2007/068982
- US-B1- 6 812 339
- PATRICK WATERS ET AL: "Multicentre comparison of a diagnostic assay: aquaporin-4 antibodies in neuromyelitis optica", JOURNAL OF NEUROLOGY NEUROSURGERY & PSYCHIATRY., vol. 87, no. 9, 25 April 2016 (2016-04-25) , pages 1005-1015, XP055334759, GB ISSN: 0022-3050, DOI: 10.1136/jnnp-2015-312601

### SEQUENCE LISTING

<110> Protagen
<120> Verfahren zur Diagnose von Neuromyelitis optica
<130> PRO139WO 2013 01
<160> 10
<170> PatentIn version 3.3
<210> 1
   <211> 323
   <212> PRT
   <213> Homo sapiens
<400> 1
   Met Ser Asp Arg Pro Thr Ala Arg Arg Trp Gly Lys Cys Gly Pro Leu
   1               5                   10                  15
   Cys Thr Arg Glu Asn Ile Met Val Ala Phe Lys Gly Val Trp Thr Gln
               20                  25                  30
   Ala Phe Trp Lys Ala Val Thr Ala Glu Phe Leu Ala Met Leu Ile Phe
           35                  40                  45
   Val Leu Leu Ser Leu Gly Ser Thr Ile Asn Trp Gly Gly Thr Glu Lys
       50                  55                  60
   Pro Leu Pro Val Asp Met Val Leu Ile Ser Leu Cys Phe Gly Leu Ser
   65                  70                  75                  80
   Ile Ala Thr Met Val Gln Cys Phe Gly His Ile Ser Gly Gly His Ile
                   85                  90                  95
   Asn Pro Ala Val Thr Val Ala Met Val Cys Thr Arg Lys Ile Ser Ile
               100                 105                 110
   Ala Lys Ser Val Phe Tyr Ile Ala Ala Gln Cys Leu Gly Ala Ile Ile
           115                 120                 125
   Gly Ala Gly Ile Leu Tyr Leu Val Thr Pro Pro Ser Val Val Gly Gly
       130                 135                 140
   Leu Gly Val Thr Met Val His Gly Asn Leu Thr Ala Gly His Gly Leu
   145                 150                 155                 160
   Leu Val Glu Leu Ile Ile Thr Phe Gln Leu Val Phe Thr Ile Phe Ala
                   165                 170                 175
   Ser Cys Asp Ser Lys Arg Thr Asp Val Thr Gly Ser Ile Ala Leu Ala
               180                 185                 190
   Ile Gly Phe Ser Val Ala Ile Gly His Leu Phe Ala Ile Asn Tyr Thr
           195                 200                 205
   Gly Ala Ser Met Asn Pro Ala Arg Ser Phe Gly Pro Ala Val Ile Met
       210                 215                 220
   Gly Asn Trp Glu Asn His Trp Ile Tyr Trp Val Gly Pro Ile Ile Gly
   225                 230                 235                 240
   Ala Val Leu Ala Gly Gly Leu Tyr Glu Tyr Val Phe Cys Pro Asp Val
                   245                 250                 255
   Glu Phe Lys Arg Arg Phe Lys Glu Ala Phe Ser Lys Ala Ala Gln Gln
               260                 265                 270
   Thr Lys Gly Ser Tyr Met Glu Val Glu Asp Asn Arg Ser Gln Val Glu
           275                 280                 285
   Thr Asp Asp Leu Ile Leu Lys Pro Gly Val Val His Val Ile Asp Val
       290                 295                 300
   Asp Arg Gly Glu Glu Lys Lys Gly Lys Asp Gln Ser Gly Glu Val Leu
   305                 310                 315                 320
   Ser Ser Val
<210> 2
   <211> 208
   <212> PRT
   <213> Artificial sequence
<220>
   <223> Part from aquaporin-4
<400> 2
   Met Ser Asp Arg Pro Thr Ala Arg Arg Trp Gly Lys Cys Gly Pro Leu
   1               5                   10                  15
   Cys Thr Arg Glu Asn Ile Met Val Ala Phe Lys Gly Val Trp Thr Gln
               20                  25                  30
   Gly Pro Gly Pro Thr Ile Asn Trp Gly Gly Thr Glu Lys Pro Leu Pro
           35                  40                  45
   Val Asp Gly Pro Gly Pro Gly Gly His Ile Asn Pro Ala Val Thr Val
       50                  55                  60
   Ala Met Val Cys Thr Arg Lys Ile Ser Gly Pro Gly Pro Leu Val Thr
   65                  70                  75                  80
   Pro Pro Ser Val Val Gly Gly Leu Gly Val Thr Met Val His Gly Asn
                   85                  90                  95
   Gly Pro Gly Pro Cys Asp Ser Lys Arg Thr Asp Val Thr Gly Ser Ile
               100                 105                 110
   Gly Pro Gly Pro Asn Pro Ala Arg Ser Phe Gly Pro Ala Val Ile Met
           115                 120                 125
   Gly Asn Trp Glu Asn His Trp Gly Pro Gly Pro Asp Val Glu Phe Lys
       130                 135                 140
   Arg Arg Phe Lys Glu Ala Phe Ser Lys Ala Ala Gln Gln Thr Lys Gly
   145                 150                 155                 160
   Ser Tyr Met Glu Val Glu Asp Asn Arg Ser Gln Val Glu Thr Asp Asp
                   165                 170                 175
   Leu Ile Leu Lys Pro Gly Val Val His Val Ile Asp Val Asp Arg Gly
               180                 185                 190
   Glu Glu Lys Lys Gly Lys Asp Gln Ser Gly Glu Val Leu Ser Ser Val
           195                 200                 205
<210> 3
   <211> 14
   <212> PRT
   <213> Artificial
<220>
   <223> Part from aquaporin-4
<400> 3
   Ser Asp Arg Pro Thr Ala Arg Arg Trp Gly Lys Cys Gly Pro
   1               5                   10
<210> 4
   <211> 10
   <212> PRT
   <213> Artificial
<220>
   <223> Part from aquaporin-4
<400> 4
   Gly Gly Thr Glu Lys Pro Leu Pro Val Asp
   1               5                   10
<210> 5
   <211> 7
   <212> PRT
   <213> Artificial
<220>
   <223> Part from aquaporin-4
<400> 5
   Cys Asp Ser Lys Arg Thr Asp
   1               5
<210> 6
   <211> 7
   <212> PRT
   <213> Artificial
<220>
   <223> Part from aquaporin-4
<400> 6
   Asn Pro Ala Arg Ser Phe Gly
   1               5
<210> 7
   <211> 14
   <212> PRT
   <213> Artificial
<220>
   <223> Part from aquaporin-4
<400> 7
   Glu Phe Lys Arg Arg Phe Lys Glu Ala Phe Ser Lys Ala Ala
   1               5                   10
<210> 8
   <211> 16
   <212> PRT
   <213> Artificial
<220>
   <223> Part from aquaporin-4
<400> 8
   Glu Phe Lys Arg Arg Phe Lys Glu Ala Phe Ser Lys Ala Ala Gln Gln
   1               5                   10                  15
<210> 9
   <211> 19
   <212> PRT
   <213> Artificial
<220>
   <223> Part from aquaporin-4
<400> 9
   Glu Phe Lys Arg Arg Phe Lys Glu Ala Phe Ser Lys Ala Ala Gln Gln
   1               5                   10                  15
   Thr Lys Gly
<210> 10
   <211> 16
   <212> PRT
   <213> Artificial
<220>
   <223> Part from aquaporin-4
<400> 10
   Asp Val Asp Arg Gly Glu Glu Lys Lys Gly Lys Asp Gln Ser Gly Glu
   1               5                   10                  15

## Beschreibung

Die vorliegende Erfindung betrifft ein Verfahren zur Diagnose und / oder Risikostratifizierung von Neuromyelitis optica (kurz: NMO), wobei eine Bestimmung aus einer Körperprobe eines Patienten/Probanden mittels artifiziellen Aquaporin-4 Peptiden erfolgt. Weiterhin betrifft die Erfindung einen Kit und neue artifizielle Aquaporin-4 Peptide als solche.

Zwecks einer geeigneten Therapie von NMO, bedarf es einer frühen Diagnose und Differenzierung von NMO, insbesondere zur Differenzierung von Multipler Sklerose (MS).

Im Stand der Technik ist das humane Aquaporin-4 als Autoantigen zum Nachweis und Diagnose von NMO beschrieben (US 20080145870 und WO 2005/051178A2 bzw. EP 1700120B1). Hierbei ist bekannt, dass im Serum von NMO-Patienten Autoantikörper nachweisbar sind, die spezifisch an das humane Aquaporin-4 binden, wie ein solches in der SEQ ID No. 1 (323 AS) abgebildet. Ein entsprechender ELISA-Kit ist auf dem Markt erhältlich (V. A. Lennon et al. A serum autoantibody marker of neuromyelitis optica: distinction from multiple sclerosis, Lancet 2C04 364(9451): 2106 - 2112; V. A. Lennon et al. IgG marker of optic-spinal multiple sclerosis binds to the aquaporin-4 water channel, The Journal of Experimental Medicine 2005 202: 473 - 477 B; G. Weinshenker et al. Neuromyelitis optica IgG predicts relapse after longitudinally extensive transverse myelitits. Annals of Neurology 2006 59: 566 - 569).

Nachteilig an den bekannten Diagnoseverfahren unter Verwendung der bisher bekannten Marker ist jedoch weiterhin, dass eine frühzeitige und vollständige Erfassung von Risikopatienten nicht ausreichend gelingt und daher eine Risikostratifizierung nicht ausreichend erfolgen kann. Eine der Erfindung zugrunde liegende Aufgabe besteht daher darin, ein Verfahren zur Diagnose und Risikostratifizierung von NMO zu entwickeln, welches eine verbesserte Erfassung von Risikopatienten ermöglicht, insbesondere im Point-of-Care Bereich.

Es ist nachteilig, dass im Stand der Technik zumeist keine hinreichende Sensitivität und/oder Spezifität mittels einem Schnelltest im Point-of-Care Bereich erreicht wird.

Daher ist es Aufgabe der vorliegenden Erfindung, ein Verfahren zur in-vitro Diagnose und / oder Risikostratifizierung von NMO bereitzustellen.

Die Aufgabe wird durch ein Verfahren zur Diagnose und Risikostratifizierung von NMO gelöst, wobei eine Bestimmung von Autoantikörpern aus einer Körperprobe mittels artifiziellen Aquaporin-4 Peptiden erfolgt (nachstehend erfindungsgemäßes Verfahren).

Überraschender Weise weisen nunmehr artifizielle Aquaporin-4 Peptide, eine hohe Sensitivität und Spezifität für die Diagnose von NMO auf.

Im Rahmen dieser Erfindung wird unter "Aquaporin-4" ein Protein verstanden, dass in der Funktion eines Wasserkanals in Zellmembranen an der Regulation der Wasser- und Elektrolyt-Balance vor allem im zentralen Nervensystem beteiligt ist. Diese Wasserkanäle sind besonders häufig in jenen Abschnitten anzutreffen, die bei der NMO klassischerweise befallen sind. Ein geeignetes Aquaporin-4 ist in der SEQ ID No. 1 mit 323 Aminosäuren abgebildet. Autoantikörper gegen das Autoantigen Aquaporin-4 werden von peripheren Plasmazellen gebildet. Nach Bindung an ihr Zielantigen führen sie zu einer Komplementaktivierung mit lokaler entzündlicher Demyelinisierung und Nekrose. Das Krankheitsbild entspricht einer Neuritis nervi optici und einer lokalen Myelitis über drei oder mehr Wirbelsäulensegmente mit vorwiegender Lokalisation an oder in der Nähe der Blut-Hirn-Schranke.

Der erfindungsgemäße Begriff "NMO" (synonym: Devic-Syndrom) betrifft eine entzündliche Autoimmunerkrankung des zentralen Nervensystems. Betroffen sind besonders das Rückenmark (Myelitis) und die Sehnerven (Neuritis nervi optici). Die Krankheit manifestiert sich mit Lähmungen der Arme und Beine, Empfindungsverlust und Störungen der Kontinenz samt Erblindung. Histologisch finden sich im betroffenen Gewebe perivasculäre Ablagerungen von Autoantikörpern-Immunglobulinen und Komplementfaktoren. Die gebildeten Autoantikörper sind spezifisch für NMO und können zur Differentialdiagnose von Multipler Sklerose mittels des Autoantigens humanen Aquaporin-4 herangezogen werden.

Im Rahmen der vorliegenden Erfindung werden artifizielle Aquaporin-4 Peptide, so wie in Anspruch 1 definiert, eingesetzt, wobei ausgehend von SEQ ID No. 1 die artifiziellen Aquaporin-4 Peptide gegenüber der SEQ ID No.1 zusätzlich mindestens einen Spacer mit 2 bis 30 Aminosäuren enthält, vorzugsweise 2 bis 20 Aminosäuren, besonders bevorzugt 2 bis 10 Aminosäuren, wobei der Spacer keine Aminosäureabfolge der SEQ ID No. 1 enthält.

In einer bevorzugten Ausführungsform der Erfindung besteht der Spacer aus hydrophilen Aminosäuren (Serin, Threonin, etc.). Weiterhin sind Helix-brechende Aminosäuren bevorzugt, wie insbesondere Prolin (P) und Glycin (G) (nachstehend: GP-Spacer).

Der Spacer kann weiterhin jede beliebige Aminosäure enthalten als auch artifizielle Aminosäuren.

Weitere geeignete artifizielle Aquaporin-4 Peptide können sein:
In einer weiteren besonderen Ausführungsform ist mindestens ein Spacer zwischen den Aminosäuren 32 und 253 der SEQ ID No. 1 eingesetzt/enthalten. Weiterhin ist bevorzugt, dass mindestens ein Spacer in den Bereichen der Transmembrandomänen eingesetzt/enthalten ist, ausgewählt aus den Sequenzabschnitten der Aminosäuren 33 bis 55, 70 bis 92, 112 bis 134, 154 bis 176, 189 bis 211 und 231 bis 253 der SEQ ID No. 1. Die Erfindung betrifft: Autoantigene zur *in-vitro* Diagnose von NMO bestehend aus einer Aminosäuresequenz (Polypetid), die folgende Teilsequenzen der Gruppen i.) SDRPTARRWGKCGP, ii.) GGTEKPLPVD, iii.) CDSKRTD, iv.) NPARSFG, und v.) DVDRGEEKKGKDQSGE, sowie eine Teilsequenz der Gruppe vi.) nämlich EFKRRFKEAFSKAA oder EFKRRFKEAFSKAAQQ oder EFKRRFKEAFSKAAQQTKG enthalten und zumindest einen Spacer aufweisen, welcher 2 bis 30 Aminosäuren enthält, vorzugsweise 2 bis 20 Aminosäuren, besonders bevorzugt 2 bis 10 Aminosäuren und das erhaltene Polypeptid aus maximal 250 Aminosäuren besteht, wobei der Spacer keine Aminosäureabfolge der SEQ ID No. 1 enthält. Eine besonders bevorzugte Ausführungsform ist SEQ ID No. 2, wobei mehrere GP-Spacer enthalten sind. Weiterhin betrifft die Erfindung die Verwendung dieser erfindungsgemäßen Autoantigene zur Diagnose von NMO nach einer der vor- oder nachstehenden Ausführungsformen.

Der Fachmann ist in der Lage entsprechende erfindungsgemäße Aminosäuresequenzen, also artifizielle Aquaporin-4 Peptide, herzustellen, z.B. vollsynthetisch mittels der Merrifield-Synthese (Weng C. Chan, Peter D. White: Fmoc Solid Phase Peptide Synthesis: A Practical Approach. Oxford University Press, Oxford/ New York 2000).

Besonders bevorzugt ist jedoch die Bereitstellung entsprechender cDNA, z.B. ausgehend von einer cDNA, wie z.B. SEQ ID No. 1 aus EP1700120B1, wobei entsprechende cDNA für die erfindungsgemäßen artifiziellen Aquaporin-4 Peptide hergestellt werden können. Entsprechende Techniken / Vorschriften sind z.B. Sambrook et al, "Molecular Cloning, A laboratory handbook, 2nd edition (1989), CSH press, Cold Spring Harbor, New York; Frederick M. Ausubel, Short protocols in molecular biology: a compendium of methods from Current protocols in molecular biology, Band 1, Wiley, 2002 zu entnehmen. Ein solcher Spacer auf DNA-Basis kann beispielsweise mittels Ligation in die cDNA eingebracht werden.

Wie bereits dargelegt sind die Spacer bevorzugt in den ausgewiesenen Bereichen der Transmembrandomänen der SEQ ID No. 1 einzubauen. Dies führt erfindungsgemäß zu einer erhöhten Sensitivität von Autoantikörpern dieser erhaltenen artifiziellen Aquaporin-4 Peptiden gegenüber dem bekannten Aquaporin-4, wahrscheinlich aufgrund des Wegfalls von Helices in der Quartärstruktur. Zudem ist dies erfindungsgemäß vorteilhaft, da eine vereinfachte gentechnische Expression der artifiziellen Aquaporin-4 Peptiden in einem Wirt, wie E. coli, ermöglicht wird.

Besonders vorteilhaft erlaubt das erfindungsgemäße Verfahren insbesondere in Fällen der Notfall- und /oder Intensivmedizin eine sichere Stratifizierung. Das erfindungsgemäße Verfahren ermöglicht daher klinische Entscheidungen, die zu einem schnellen Therapieerfolg und zur Vermeidung von Todesfällen führen. Solche klinischen Entscheidungen umfassen ebenfalls weiterführende Behandlung mittels Arzneimitteln zur Behandlung oder Therapie von NMO.

Daher betrifft die Erfindung ebenfalls ein Verfahren zur Diagnose und / oder Risikostratifizierung von NMO-Patienten zur Durchführung von klinischen Entscheidungen, wie weiterführende Behandlung und Therapie mittels Arzneimitteln, vorzugsweise in der zeitlich kritischen Intensivmedizin oder Notfallmedizin, einschließlich der Entscheidung zur Hospitalisierung des Patienten.

In einer weiteren bevorzugten Ausführungsform betrifft das erfindungsgemäße Verfahren daher die Therapiesteuerung von NMO.

In einer weiteren bevorzugten Ausführungsform des erfindungsgemäßen Verfahrens erfolgt die Diagnose und / oder Risikostratifizierung zur Prognose, zur differentialdiagnostischen Früherkennung und Erkennung, zur Beurteilung des Schweregrades und zur therapiebegleitenden Verlaufsbeurteilung.

In einer weiteren bevorzugten Ausführungsform betrifft die Erfindung ein Verfahren zur in-vitro Diagnostik zur Früh- oder Differentialdiagnose oder Prognose von NMO, wobei eine Bestimmung von Autoantikörpern aus einer Körperprobe mittels artifiziellen Aquaporin-4 Peptiden an oder von einem zu untersuchenden Patienten durchgeführt wird.

In einer Ausführungsform des erfindungsgemäßen Verfahrens wird dem zu untersuchenden Patienten eine Körperprobe, insbesondere Gewebe oder Körperflüssigkeit, vorzugsweise Blut entnommen, wahlweise Vollblut oder Serum oder erhältliches Plasma und die Diagnose erfolgt *in vitro*/*ex vivo,* d.h. außerhalb des menschlichen oder tierischen Körpers. Aufgrund der Bestimmung von Autoantikörpern aus einer Körperprobe mittels artifiziellen Aquaporin-4 Peptiden, wird eine hohe Sensitivität und Spezifität erzielt und anhand der vorhandenen Menge in mindestens einer Patientenprobe kann die Diagnose oder Risikostratifizierung erfolgen.

Die Menge an Autoantikörpern kann beispielsweise mittels radioaktivmarkierten oder biotinylierten artifiziellen Aquaporin-4 Peptiden erfolgen (z.B. S(35)-Methode oder dergleichen). Freibleibende artifizielle Aquaporin-4 Peptide können z.B. extrahiert werden. Weiterhin können an artifiziellen Aquaporin-4 Peptiden gebundene Autoantikörper mittels Anti-Autoantikörper fluoresenztechnisch nachgewiesen werden.

In einer weiteren Ausführungsform der Erfindung kann das erfindungsgemäße Verfahren im Rahmen einer in-vitro Diagnose mittels parallelen oder simultanen Bestimmungen mittels der artifiziellen Aquaporin-4 Peptiden durchgeführt werden (z.B. Multititerplatten mit 96 und mehr Kavitäten), wobei die Bestimmungen an mindestens einer Patientenprobe durchgeführt werden.

In einer weiteren Ausführungsform kann das erfindungsgemäße Verfahren und dessen Bestimmungen mittels einem Schnelltest durchgeführt werden (z.B. lateral-flow Test oder Point-of Care), sei es in Einzel- oder Multiparameterbestimmung. In einer besonders bevorzugten Ausführungsform handelt es sich um einen Selbsttest oder um eine Vorrichtung, die in der Notfalldiagnostik geeignet ist. Eine weitere Aufgabe ist die Bereitstellung einer entsprechenden diagnostischen Vorrichtung oder deren Verwendung zur Durchführung des erfindungsgemäßen Verfahrens. Im Rahmen dieser Erfindung wird unter einer solchen diagnostischen Vorrichtung, insbesondere ein Array oder Assay verstanden (z.B. Immunoassay, ELISA etc.), im weitesten Sinne eine Vorrichtung zur Durchführung des erfindungsgemäßen Verfahrens.

Der Nachweis und die Quantifizierung von gebundenen Autoimmunantikörpern an den erfindungsgemäßen artifiziellen Aquaporin-4 Peptiden kann ebenfalls mit Hilfe weiterer, dem Fachmann geläufigen Protein-Diagnoseverfahren durchgeführt werden, insbesondere unter Verwendung radioaktiv oder enzym-bzw. fluoreszenzmarkierter Antikörper. Zu nennen sind insbesondere dazu geeignete bioanalytische Verfahren, wie zum Beispiel Immunhistochemie, Antikörperarrays, Luminex, ELISA, Immunfluoreszenz, Radioimmunoassays sowie weiteren geeigneten bioanalytischen Verfahren, wie zum Beispiel massenspektrometrischen Verfahren, z.B. MRM (Multi reaction monitoring) oder AQUA (absolute Quantification), mit deren Hilfe die Autoimmunantikörper quantitativ gemessen und der jeweilige Nachweis durchgeführt werden kann.

Die Erfindung betrifft zudem ein Kit zur Diagnose und / oder Risikostratifizierung von NMO, enthaltend artifizielle Aquaporin-4 Peptide samt Nachweisreagenzien und ggfs. Hilfsmittel, wie in Anspruch 4 definiert. Solche Nachweisreagenzien umfassen z.B. Antikörper, Beads etc.

Nachfolgende Beispiele und Figuren dienen zur näheren Erläuterung der Erfindung, jedoch ohne die Erfindung auf diese Beispiele und Figuren zu beschränken.

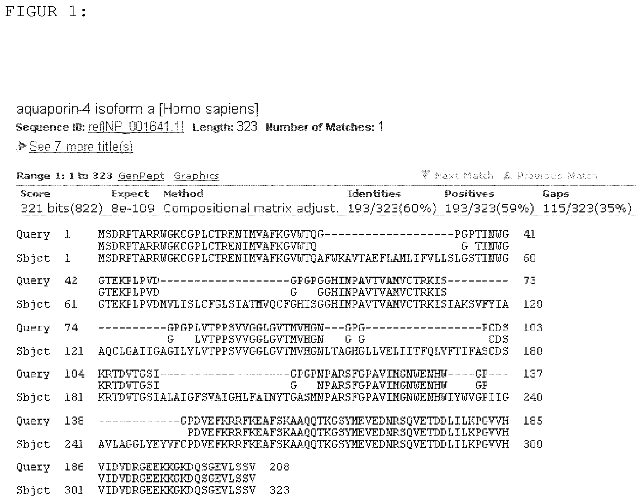

Figur 1 zeigt die strukturellen Unterschiede zwischen SEQ ID No. 1 (humanes Aquaporin-4) und der erfindungsgemäßen SEQ ID No. 2, enthaltend GP-Spacer samt den bevorzugten Teilsequenzen.

### Beispiele:

Das erfindungsgemäße Verfahren kann z.B. analog gemäß Lennon 2004, 2005 (supra) durchgeführt werden.

In einer Mikrotiterplatte (96 und mehr Wells) werden bei Raumtemperatur Kalibratoren, teilweise Positivkontrollen, teilweise Negativkontrollen eingegeben.

Eine erfindungsgemäße artifizielles Aquaporin-4 gemäß SEQ ID No. 2 wird als Autoantigen eingesetzt, z.B. biotinyliert oder radioaktiv markiert und in Puffer gelöst (z.B. PBS) und gerührt.

Im Fall von biotinylierten artifiziellen Aquaporin-4 gemäß SEQ ID No. 2 kann ein Autoantikörperbindungsnachweis mittels Streptavidin Peroxidase erfolgen (siehe z.B. Prescott, L. M.; Harley, J. P.; Klein, D. A.: Microbiology. 5th Edition. McGraw Hill, Boston 2002).

50 µl NMO-Patientenserum werden in einer Mikrotiterplatte, jeweils mit Kontrollen und Kalibratoren versetzt.

Es werden 25 µl einer Lösung aus biotinyliertem artifiziellen Aquaporin-4 gemäß SEQ ID No. 2 in jedes Well eingegeben.

Der in dieser Weise präparierte ELISA wird dreimal mit einer Waschlösung gewaschen. Anschließend werden die Wells mit 100 µl Streptavidin Peroxidase versetzt und geschüttelt, gewaschen und mit einem Peroxidase Substrat versetzt und inkubiert. Danach erfolgt eine photometrische Auswertung anhand einer Kalibrierungskurve.

Das erfindungsgemäße Verfahren kann ebenfalls mit einem üblichen Radioimmunassay (RIA) durchgeführt werden.

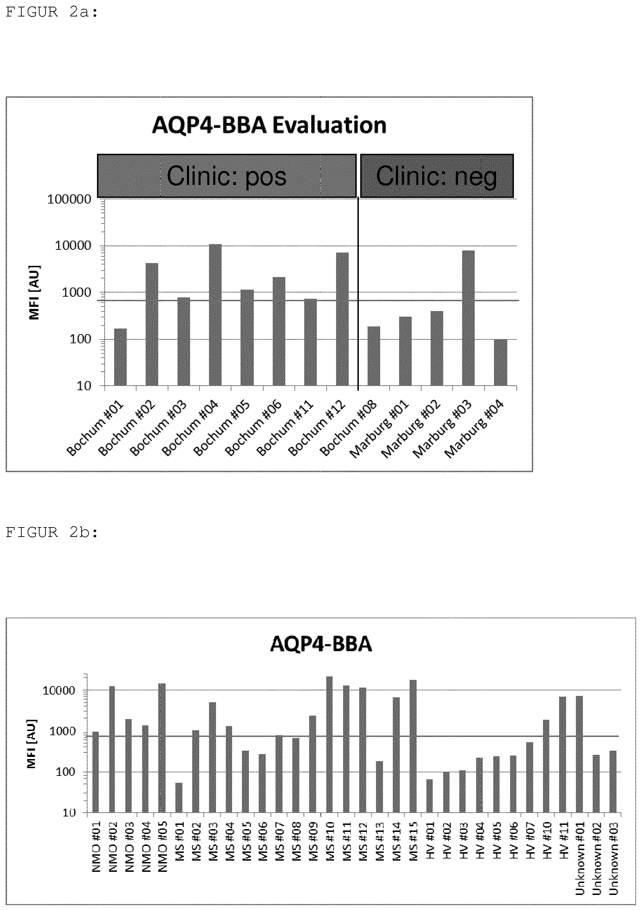

Figur 2a zeigt die Diagnose von NMO anhand 8 positiver und 5 negativer Patientenproben (Klinik) mittels einem Luminex Bead Assay unter Verwendung von SEQ ID No. 2, wobei bei einem ermittelten Schwellenwert von 675 MFI (median fluorescence intensity) 85 % der klinischen Patienten richtig getestet werden konnten.

Figur 2b zeigt die Stratifizierung von NMO-Patienten ("NMO") gegen Multiple Sklerose Patienten (MS) und gesunden Patienten (HV) mittels einem Luminex Bead Assay unter Verwendung von SEQ ID No. 2, wobei bei einem ermittelten Schwellenwert von 675 MFI NMO-Patienten zu 100 % wiedergefunden wurden.

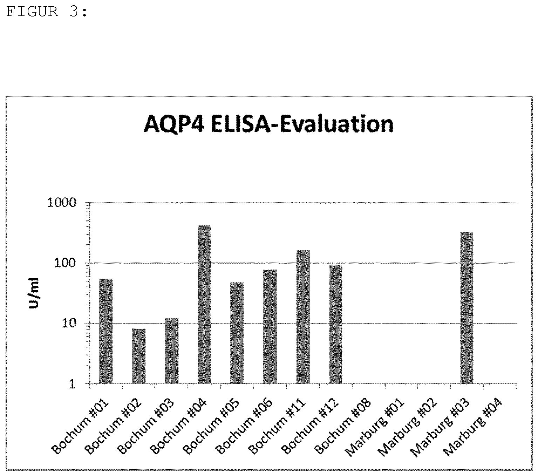

Figur 3a zeigt die Diagnose von NMO anhand 9 positiver und 4 negativer Patientenproben (Klinik) mittels einem ELISA Assay gemäß o.g. Beispiel unter Verwendung von SEQ ID No. 2, wobei 92 % der klinischen Patienten richtig getestet werden konnten.

## Patentansprüche

1. Autoantigene zur in-vitro Diagnose von NMO bestehend aus einer Aminosäuresequenz (Polypetid), die folgende Teilsequenzen der Gruppen i.) SDRPTARRWGKCGP, ii.) GGTEKPLPVD, iii.) CDSKRTD, iv.) NPARSFG, und v.) DVDRGEEKKGKDQSGE, sowie eine Teilsequenz der Gruppe vi.) nämlich EFKRRFKEAFSKAA oder EFKRRFKEAFSKAAQQ oder EFKRRFKEAFSKAAQQTKG enthalten
und
zumindest einen Spacer aufweisen, welcher 2 bis 30 Aminosäuren enthält, vorzugsweise 2 bis 20 Aminosäuren, besonders bevorzugt 2 bis 10 Aminosäuren und das erhaltene Polypeptid aus maximal 250 Aminosäuren besteht, wobei der Spacer keine Aminosäureabfolge der SEQ ID No. 1 enthält.

2. Autoantigen bestehend aus der Aminosäuresequenz gemäß SEQ ID No. 2.

3. Autoantigene zur in-vitro Diagnose von NMO bestehend aus einer Aminosäuresequenz (Polypetid) nach Anspruch 1, wobei der Spacer hydrophile Aminosäuren und / oder Helix-brechende Aminosäuren aufweist, insbesondere Prolin und Glycin.

4. Kit zur in-vitro Diagnose und / oder Risikostratifizierung von Neuromyelitis optica enthaltend artifizielle Aquaporin-4 Peptide nach Anspruch 1 oder Anspruch 2 samt Nachweisreagenzien und ggfs. Hilfsmittel.

## Claims

1. Autoantigens for *in vitro* diagnosis of NMO (neuromyelitis optica) consisting of an amino acid sequence (polypeptide), which contain the following partial sequences of groups i.) SDRPTARRWGKCGP, ii.) GGTEKPLPVD, iii.) CDSKRTD, iv.) NPARSFG, and v.) DVDRGEEKKGKDQSGE, and a partial sequence of group vi.) specifically EFKRRFKEAFSKAA or EFKRRFKEAFSKAAQQ or EFKRRFKEAFSKAAQQTKG
and
comprise at least one spacer which contains 2 to 30 amino acids, preferably 2 to 20 amino acids, particularly preferably 2 to 10 amino acids, and the polypeptide that is obtained consists of at most 250 amino acids, wherein the spacer does not contain any amino acid sequence of SEQ ID No. 1.

2. An autoantigen consisting of the amino acid sequence according to SEQ ID No. 2.

3. Autoantigens for *in vitro* diagnosis of NMO consisting of an amino acid sequence (polypeptide) according to claim 1, wherein the spacer comprises hydrophilic amino acids and/or helix-breaking amino acids, in particular proline and glycine.

4. A kit for *in vitro* diagnosis and/or risk stratification of neuromyelitis optica, containing artificial aquaporin-4 peptides according to claim 1 or claim 2, and also detection reagents and, optionally, auxiliary agents.

## Revendications

1. Autoantigènes destinés au diagnostic in-vitro de la neuromyélite optique NMO, constitués d'une séquence d'acides aminés (polypeptide), qui contiennent les séquences partielles suivantes des groupes i.) SDRPTARRWGKCGP, ii.) GGTEKPLPVD, iii.) CDSKRTD, iv.) NPARSFG, et v.) DVDRGEEKKGKDQSGE, ainsi qu'une séquence partielle du groupe vi.), à savoir EFKRRFKEAFSKAA ou EFKRRFKEAFSKAAQQ ou EFKRRFKEAFSKAAQQTKG
et
présentent au moins une séquence spacer, laquelle contient de 2 à 30 acides aminés, de préférence de 2 à 20 acides aminés, de manière particulièrement préférée de 2 à 10 acides aminés et le polypeptide obtenu est constitué d'au maximum 250 acides aminés, où la séquence spacer ne contient aucune suite d'acides aminés de la SEQ ID N ° 1.

2. Autoantigènes constitués de la séquence d'acides aminés selon SEQ ID N ° 2.

3. Autoantigènes destinés au diagnostic in-vitro de NMO constitués d'une séquence d'acides aminés (polypeptide) selon la revendication 1, dans lesquels la séquence spacer présente des acides aminés hydrophiles et / ou des acides aminés de rupture d'hélice, notamment de la proline et de la glycine.

4. Kit destiné au diagnostic in-vitro et / ou à la stratification de risques de neuromyélite optique contenant des peptides d'aquaporine-4 selon la revendication 1 ou la revendication 2, y compris des réactifs indicateurs et éventuellement des produits auxiliaires.
